**Europäisches Patentamt**

**European Patent Office**

**Office européen des brevets**

(19)

(11) Veröffentlichungsnummer: **0 146 107**
**A2**

(12) # EUROPÄISCHE PATENTANMELDUNG

(21) Anmeldenummer: **84115123.6**

(22) Anmeldetag: **11.12.84**

(51) Int. Cl.⁴: **C 12 P 21/02**

(30) Priorität: **13.12.83 HU 423783**

(43) Veröffentlichungstag der Anmeldung: **26.06.85**
**Patentblatt 85/26**

(84) Benannte Vertragsstaaten: **AT BE CH DE FR GB IT LI NL SE**

(71) Anmelder: **EGYT GYOGYSZERVEGYESZETI GYAR, Kereszturi ut 30-38, Budapest X (HU)**

(72) Erfinder: **Toth, Miklos, Dr., 314 ep. Zsitva s. 4/a, Szeged, Rokus (HU)**
Erfinder: **Endresz, Valeria, Kinizsi u. 7, Adona (HU)**
Erfinder: **Beladi, Ilona, Dr., Dugonics u. 3, Szeged (HU)**
Erfinder: **Toth, Sandor, Szücs u. 13, Szeged (HU)**

(74) Vertreter: **Hansen, Bernd, Dr.rer.nat. et al, Hoffmann, Eitle & Partner Patentanwälte Arabellastrasse 4, D-8000 München 81 (DE)**

(54) Verfahren zur nacheinanderfolgenden Herstellung von humanem Leukozyteninterferon und humanem Gamma-Interferon.

(57) Die Erfindung betrifft ein Verfahren zur Herstellung von α- und γ-Interferon durch Trennung der Leukozytenfraktion (buffy coat) des Blutes, Entfernung der Erythrozyten, Suspendieren der Leukozyten in einer geeigneten Nährlösung und Behandlung mit einem α-Interferon-Induktor und einem γ-Interferon-Induktor, dadurch gekennzeichnet, daß man das α-Interferon und γ-Interferon in nacheinanderfolgenden Stufen in derselben Leukozytenkultur erzeugt, indem man die nach Trennung der Leukozytenfraktion des Blutes, Entfernung der Erythrozyten und Suspendieren der Leukozyten in einer geeigneten Nährlösung erhaltene Suspension mit α- oder β-Interferon vorbehandelt, mit einem α-Interferon-Induktor in Berührung bringt, die α-interferonhaltige Flüssigkeit von den Zellen abtrennt, das α-Interferon erwünschtenfalls gewinnt, die Zellen wäscht und in einer geeigneten Nährlösung suspendiert, mit einem mitogenen Mittel behandelt, die γ-interferonhaltige Flüssigkeit von den Zellen abtrennt und erwünschtenfalls das γ-Interferon gewinnt und erwünschtenfalls α-interferonfrei macht.

Der Vorteil des erfindungsgemäßen Verfahrens liegt darin, daß α- und β-Interferonproduktion in derselben Zellenkultur in zwei nacheinanderfolgenden Stufen durchgeführt werden kann. Das Verfahren wird dadurch viel wirtschaftlicher und die Erfindung ermöglicht eine wesentliche Erhöhung der interferonproduzierenden Kapazität der nur begrenzt zur Verfügung stehenden Leukozyten.

- 1 -

Verfahren zur nacheinanderfolgenden Herstellung von humanem Leukozyteninterferon und humanem Gamma-Interferon

Die Erfindung betrifft ein Verfahren zur nacheinanderfolgenden Herstellung von humanem Leukozyteninterferon und humanem Gamma-Interferon.

Die vorliegende Erfindung ermöglicht die Herstellung von $\alpha$- und $\gamma$-Interferon mit der selben Zellenpopulation in zwei Stufen und dadurch wird die interferonproduzierende Kapazität der nur in begrenzten Mengen zur Verfügung stehenden humanen Leukozyten auf eine optimale und maximale Weise ausgenützt.

Es ist bekannt, dass aus humanen Leukozyten mit Viren $\alpha$-Interferon und mit Hilfe von mitogenen Mitteln $\gamma$-Interferon erzeugt werden kann. Der Herstellbarkeit der beiden Interferontypen steht die Tatsache im Wege, dass humane Leukozyten nur in begrenzter Menge zur Verfügung stehen. In der humanen Therapie werden beide Interferontypen benötigt, weil die einzelnen Interferontypen verschiedene günstige Eigenschaften besitzen (das $\alpha$-Interferon übt besonders eine antivirale Wirkung und das $\gamma$-Interferon in erster Reihe eine Antitumor-Wirkung aus) und die verschiedenen Interferontypen, wenn kombiniert verwendet, ihre Wirksamkeit potenzieren (antivirale, antitumor und immunomodulante Wirkung). Mit Rücksicht darauf, dass sowohl das $\alpha$-Interferon als auch das $\gamma$-Interferon aus den nur in begrenzten Mengen zur Verfügung stehenden Leukozyten erhalten werden, schliesst die Produktion eines Interferontyps die Erzeugung des anderen aus. Das Ziel der Erfindung ist die nacheinanderfolgende Herstellung von $\alpha$- und $\gamma$-Interferon aus der selben Leukozytenkultur. Man ist seit langem bestrebt, $\alpha$-Interferon mit der selben Zellenpopulation mehrmals nacheinander herzustellen. Diese Versuche scheiterten deshalb, weil das durch die Zellen in grossen Mengen produzierte $\alpha$-Interferon auf die Zellen rückwirkt und die Zellen in einen "hyporeaktiven Zustand" bringt, was eine drastische Herabsetzung der $\alpha$-interferonproduzierenden Kapazität der Zellen bei der nächsten Induktion zur Folge hat.

Die Erfindung beruht auf der Erkenntnis, dass das $\gamma$-Interferon produzierende System auch nach Behandlung mit einer grossen Menge von $\alpha$-Interferon nicht in einen hyporeaktiven Zustand gebracht wird und sogar mehr Interferon erzeugt als bei dem unbehandelten Muster. Dies wird auch am Ende der $\alpha$-Interferon produzierenden Periode beobachtet, so dass schliesslich eine grosse Menge von $\alpha$-Interferon erhalten wird, wobei die interferonproduzierende Kapazität der $\gamma$-Interferon erzeugenden Zellen entweder unverändert bleibt oder sogar erhöht wird.

Gegenstand der Erfindung ist ein Verfahren zur Herstellung von $\alpha$- und $\gamma$-Interferon durch Trennung der Leukozytenfraktion (buffy coat) des Blutes, Entfernung der Erythrozyten, Suspendieren der Leukozyten in einer geeigneten Nährlösung und Behandlung mit einem $\alpha$-Interferon-Induktor und einem $\gamma$-Interferon-Induktor dadurch gekennzeichnet, dass man das $\alpha$-Interferon und $\gamma$-Interferon in nacheinanderfolgenden Stufen in derselben Leukozytenkultur erzeugt, indem man die nach Trennung der Leukozytenfraktion des Blutes, Entfernung der Erythrozyten und Suspendieren der Leukozyten in einer geeigneten Nährlösung erhaltene Suspension mit $\alpha$ - oder ß-Interferon vorbehandelt, mit einem $\alpha$-Interferon-Induktor in Berührung bringt, die $\alpha$-interferonhaltige Flüssigkeit von den Zellen abtrennt, das $\alpha$-Interferon erwünschtenfalls gewinnt, die Zellen wäscht und in einer geeigneten Nährlösung suspendiert, mit einem mitogenen Mittel behandelt, die $\gamma$-interferonhaltige Flüssigkeit von den Zellen abtrennt und erwünschtenfalls das $\gamma$-Interferon gewinnt und erwünschtenfalls $\alpha$-interferonfrei macht.

Nach dem erfindungsgemässen Verfahren werden als Ausgangsstoff mit einem gerinnungshemmenden Mittel behandelte und aus menschlichem Blut, welches nicht länger als 48 Stunden lang bei 0-8°C gelagert wurde, gewonnene Leukozyten (buffy coat) eingesetzt.

Als gerinnungshemmendes Mittel kann z.B. ACD-Lösung (eine Zitronensäure und Dextrose enthaltende Lösung) oder durch verschiedene Basen (wie Adenin oder Guanin) ergänzte ACD-Lösung verwendet werden. Die gesammelten Leukozyten können durch Gradientzentrifugieren (z.B. mit Hilfe von Ficoll oder Percoll) oder vorteilhaft durch Haemolyse mit Ammoniumchlorid entfernt werden. Man kann vorteilhaft so verfahren, dass man die konzentrierte Leukozytensuspension mit einer 0,5-10 %-igen - vorzugsweise 0,83 %-igen - Ammoniumchloridlösung (0-10 °C) in einem Volumenverhältnis von 1:3-20 - vorteilhaft 1:5 - vermischt. Die Suspension kann 5-20 Minuten lang - zweckmässig etwa 10 Minuten lang - bei einer Temperatur von 0-8 °C mit oder ohne Rühren inkubiert werden. Die Leukozyten werden von den zerfallenen Erythrozyten z.B. durch Zentrifugieren abgetrennt. Die Ammoniumchloridbehandlung wird vorzugsweise so wiederholt, dass auf 1 Vol.-Teil Zellensuspension berechnet 10 Vol.-Teile Ammoniumchloridlösung verwendet werden.

Die gereinigten Leukozyten werden in einer, Aminosäuren und Vitamine enthaltenden Zellenkulturnährlösung inkubiert (z.B. Eagle-Nährlösung, RPMI 1640, Dulbecco modifizierte MEM, Glasgow modifizierte MEM usw.). Eine Nährlösung der nachstehenden Zusammensetzung lässt sich ebenfalls günstig verwenden (dieser Nährboden ist billig und kann autoklavisiert werden).

| Komponente | Menge, mg/l |
|---|---|
| Calciumchlorid | 175-350 |
| Kaliumchlorid | 300-500 |
| Magnesiumsulfat oder eine äquivalente Menge von Magnesiumchlorid | 175-500 |
| Natriumchlorid | 5000-7000 |
| Natriumbicarbonat | 200-3500 |
| Natriumdihydrogenphosphat | 30-150 |
| Glukose | 500-5500 |
| Eisen-[III]-nitrat | 0,0-0,2 |

Die Zellnummer wird auf einen Wert zwischen $10^6$ und $10^8$ - vorteilhaft $10^7$ Zellen/ml - eingestellt.

Die verwendete Nährlösung bzw. der Nährboden wird mit einem tierischen oder menschlichen Serum oder gamma-Globulin-freiem Serum ergänzt (0,5-10 %). Der Nährlösung bzw. dem Nährboden können Antibiotika (vorteilhaft Neomycin oder Gentamycin) zugegeben werden.

Die Zellen werden danach einer Behandlung mit $\alpha$ - oder ß-Interferon unterworfen. Zu diesem Zweck kann induktorfreies rohes oder gereinigtes Interferon, vorzugsweise mit einer Konzentration von 10-500, vorteilhaft 200-300 IE/ml, verwendet werden. Die Temperatur der Vorbehandlung liegt zwischen 35 und 39°C und beträgt vorzugsweise 37°C. Die Dauer der Behandlung ist 1-6 Stunden - vorteilhaft 2 Stunden.

Die Zellen werden mit einem $\alpha$-Interferon-Induktor in Berührung gebracht. Als $\alpha$-Interferon-Induktor kann vorzugsweise roher oder gereinigter Sendai-Virus eingesetzt werden. Dieser Virus wird zweckmässig in einer Konzentration von 100-800 - vorteilhaft 400 hemagglutinierende Einheiten/ml - verwendet.

Die Induktion kann 5-48 Stunden lang - vorteilhaft in 15-20 Stunden - bei einer Temperatur von 35-39°C - vorzugsweise bei 37°C - durchgeführt werden. Die Zellen werden von der flüssigen Phase getrennt. Die überstehende Schicht enthält das rohe $\alpha$-Interferon, welches bei einer Temperatur zwischen -20 und +4°C gelagert oder auf an sich bekannte Weise gereinigt werden kann.

Die Zellen werden gewaschen und zwar vorteilhaft mit einer physiologischen Salzlösung, insbesondere mit einer Nährlösung der oben angegebenen Zusammensetzung. Nach dem Waschen wird die Zellenkonzentration auf $5 \cdot 10^6 - 10^8$ - vorteilhaft auf $2,5 \cdot 10^7$ - eingestellt und zwar in einer Nährlösung oder Nährboden, vorteilhaft in einer Nährlösung der obigen Zusammensetzung. Die Nährlösung kann ein menschliches oder tierisches Serum bzw. globulinfreies Serum - vorteilhaft humanes globulinfreies Serum - in einer Menge von 0,5-5 mg/ml (vorteilhaft 1-2 mg/ml) enthalten (3-6 %).

Die Zellen werden mit einem $\gamma$-Interferon-Inducer behandelt. Zu diesem Zweck haben sich Concanavalin A, Phytohemagglutinin und Staphylococcus enterotoxin als besonders vorteilhaft erwiesen. Nach einer vorteilhaften Ausführungsform des erfindungsgemässen Verfahrens wird als Inducer (Induktor) Concanavalin A verwendet und zwar in einer Konzentration von 2,5-30 µg/ml, vorteilhaft 15 µg/ml.

Die Induktion kann 8-48 Stunden lang - vorteilhaft in 12-16 Stunden - bei einer Temperatur zwischen 35 und 39°C - vorzugsweise bei 37°C - durchgeführt werden. Nach einer bestimmten Zeit (etwa eine Stunde) kann der Induktor erwünschtenfalls durch Waschen entfernt werden. Diese Massnahme kann jedoch auch weggelassen werden, weil die Anwesenheit des Induktors keine negative Wirkung auf die Produktion des $\gamma$-Interferons hat. Der Induktor wird im allgemeinen nicht entfernt. Die Zellen werden von der flüssigen Phase getrennt. Die überstehende Flüssigkeit enthält das rohe $\gamma$-Interferon. Dieses Produkt ist mit $\alpha$-Interferon verunreinigt, weil in der zweiten Periode der Interferonproduktion (d.h. in der Periode der $\gamma$-Interferonerzeugung) die $\alpha$-Interferon erzeugenden Zellen noch mehr oder weniger Interferon produzieren.

Mit Rücksicht darauf, dass die verschiedenen Interferontypen ihre antivirale Wirkung potenzieren, kann der "tatsächliche $\gamma$-Interferon-Gehalt" der $\alpha$- und

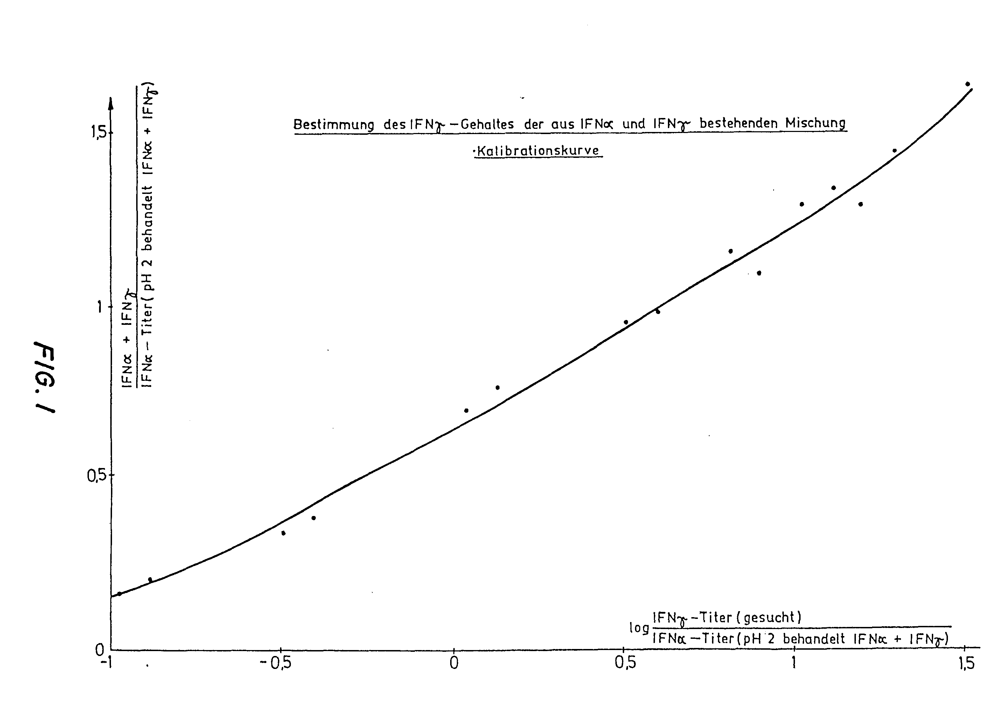

$\gamma$-Interferon enthaltenden Mischungen nur mit Hilfe einer Kalibrationskurve berechnet werden (Fig. 1). Zur Berechnung der Menge des $\gamma$-Interferons muss der potentierte Titer und $\alpha$-Interferon-Gehalt der Mischung bekannt sein. Der letztere Wert kann so bestimmt werden, dass man die Mischung auf einen pH Wert von 2 bringt und danach titriert; bei diesem pH-Wert wird nämlich das $\gamma$-Interferon auf eine selektive Weise zersetzt.

Das reine $\gamma$-Interferon wird so erhalten, dass man das rohe $\gamma$-Interferon $\alpha$-interferonfrei macht. Die Entfernung des $\alpha$-Interferons wird vorteilhaft nach partieller Reinigung und Konzentrierung des rohen $\gamma$-Interferons (z.B. an CPG-350 porösen Glasteilchen, Electro Nucleonics N.J. USA) durchgeführt. Die Entfernung des $\alpha$-Interferons kann nach verschiedenen Methoden durchgeführt werden. Eine Methode beruht auf den verschiedenen Molekulargewichten der beiden Interferone ( $\alpha$ = 18,000-21,000 Dalton; gamma = = 40,000-45,000 Dalton) und wird mit Hilfe von Gelfiltrieren durchgeführt (z.B. Sephacryl S-200 Chromatographie). Nach der anderen Methode wird die $\alpha$-Interferon-Verunreinigung mit an Sepharose-Gel verbundenen anti--$\alpha$-Interferon-Antikörpern entfernt.

Der Vorteil des erfindungsgemässen Verfahrens liegt darin, dass $\alpha$- und $\gamma$-Interferon in der selben Leukozytenkultur nacheinander in zwei Stufen erzeugt werden können. Die Produktionskosten - auf 1 Einheit Interferon berechnet - werden dadurch erheblich niedriger, weil der grössere Teil der Interferonherstellungskosten auf die Sammlung der Blutproben, die Bereitung der "buffy coat" Fraktion und die Reinigung der Leukozyten fällt. Mit Hilfe des erfindungsgemässen Verfahrens wird die interferonproduzierende Kapazität der nur begrenzt zur Verfügung stehenden Leukozyten wesentlich erhöht.

Weitere Einzelheiten des erfindungsgemässen Verfahrens sind den nachstehenden Beispielen zu entnehmen, ohne den Schutzumfang auf diese Beispiele einzuschränken.


## Beispiel 1

Das in eine ACD-Lösung ( Zitronensäure und Dextrose enthaltende wässrige Lösung) entnommene Blut wird 3 Stunden lang bei +4 $^\circ$C gelagert. 1 Vol.-Teil des so erhaltenen Leukozytenkonzentrats wird mit 5 Vol.- -Teilen einer 0,83 %-igen wässrigen eiskalten Ammoniumchloridlösung vermischt. Die Suspension wird so lange unter Eis gehalten, bis die Lysierung der Erythrozyten stattfindet (5-10 Minuten). Danach werden die Leukozyten in einer Nährlösung folgender Zusammensetzung suspendiert:

| Komponente | Menge, mg/l |
|---|---|
| Calciumchlorid | 175-350 |
| Kaliumchlorid | 300-500 |
| Magnesiumsulfat oder eine äquivalente Menge von Magnesiumchlorid | 175-500 |
| Natriumchlorid | 5000-7000 |
| Natriumbicarbonat | 200-3500 |
| Natriumdihydrogenphosphat | 30-150 |
| Glukose | 500-5500 |
| Eisen.-/III/-nitrat | 0,0-0,2 |

Die obige Lysierung der Erythrozyten wird so wiederholt, dass man auf 1 Vol.-Teil der Leukozytensuspension berechnet 10 Vol.-Teile einer 0,83 %-igen
wässrigen Ammoniumchloridlösung verwendet. Die
Leukozyten werden in der obigen Nährlösung suspendiert,
worauf die Zellenzahl auf $10^7$ Zellen/ml eingestellt wird.

Die Nährlösung enthält 2 mg/ml humanes globulinfreies Serum (etwa 6 %). Die Zellen werden bei 37 $^{\circ}$C
unter ständigem Rühren mit 200 IE/ml induktorfreiem
konzentriertem humanem $\alpha$-Interferon behandelt. Nach
2 Stunden werden die Zellen mit 400 hemagglutinierenden
Einheiten Sendai Viren induziert. Die Inkubation wird in
der 18. Stunde nach der Induktion beendigt. Der antivirale Titer der supernatierenden Flüssigkeit - d.h.
des rohen $\alpha$-Interferons - beträgt 54,200 IE/ml. Die zur
Erzeugung des $\alpha$-Interferons verwendeten Zellen werden
mit der obigen Nährlösung einmal gewaschen, wonach die
Leukozyten in der Nährlösung in einer Konzentration von
2,5 · $10^7$ Zellen/ml suspendiert werden. Die Nährlösung
enthält 1 mg/ml globulinfreies humanes Serum (etwa 3 %).
Danach werden die Zellen mit 15 µg/ml Concanavalin A
stimuliert. Der Induktor wird aus dem System nicht
entfernt. Die Inkubation wird unter ständigem Rühren bei
37 $^{\circ}$C durchgeführt, und in der 16. Stunde nach der
Induktion wird die supernatierende Flüssigkeit von den
Zellen getrennt. Der antivirale Titer der
supernatierende Flüssigkeit, welche das $\gamma$-Interferon
enthält, wird an WISH humanen amnialen Zellen bestimmt,
und die Titer werden nach Vergleich mit einem humanen
$\alpha$-Interferon Standard als Einheiten/ml (E/ml) ausgedrückt. (Es liegt nämlich gegenwärtig noch kein
international anerkannter Interferonstandard vor). Der
Titer der supernatierenden Flüssigkeit beträgt 10,600 E/ml; dies ist jedoch ein potenzierter Wert, weil die γ-Interferon Präparate auch α-Interferon enthalten. Mit Hilfe der auf der Abbildung gezeigten Kalibrationskurve und auf Grund der Titer der ursprünglichen und bei einem pH-Wert von 2 behandelten Substanzen beträgt der "tatsächliche" γ-Interferongehalt 1,340 E/ml.

Zum Vergleich wird das obige Verfahren mit der Änderung durchgeführt, dass die Zellen vor der γ-Interferon--Erzeugung ohne Behandlung mit Sendai-Virus einen Tag lang inkubiert werden. Der unter Einwirkung von Concanavalin A erzeugte γ-Interferontiter beträgt 340 E/ml.

Zum weiteren Vergleich wird die erste - d.h. α-Interferon produzierende - Periode weggelassen und die isolierten Leukozyten werden unmittelbar mit Concanavalin A induziert. Der γ-Interferontiter beträgt 450 E/ml.

Werden die Leukozyten mit Weglassung der α-Interferonerzeugungsperiode vor der Induktion 4 Stunden lang mit 1,500 IE/ml α- oder β-Interferon behandelt (priming), beträgt der Titer des erzeugten γ-Interferons 2,300 E/ml.

## Beispiel 2

Man verfährt wie im Beispiel 1, mit dem Unterschied, dass man als Nährlösung eine Eagle-Nährlösung verwendet /¯Virology <u>14</u>, 359 /1961/_7. Der so erhaltene α-Interferontiter beträgt 56,000 IE/ml. In der zweiten Stufe des Verfahrens wird in der selben Nährlösung ein γ-Interferontiter von 1,680 E/ml erreicht.

Wird nach diesem Verfahren die α-Interferonproduktion weggelassen, beträgt der γ-Interferontiter 280 E/ml.

Patentansprüche

1. Verfahren zur Herstellung von α- und γ-Interferon durch Trennung der Leukozytenfraktion (buffy coat) des Blutes, Entfernung der Erythrozyten, Suspendieren der Leukozyten in einer geeigneten Nährlösung und Behandlung mit einem α-Interferon-Induktor und einem γ-Interferon-Induktor d a d u r c h g e k e n n z e i c h n e t , dass man das α-Interferon und γ-Interferon in nacheinanderfolgenden Stufen in der selben Leukozytenkultur erzeugt, indem man die nach Trennung der Leukozytenfraktion des Blutes, Entfernung der Erythrozyten und Suspendieren der Leukozyten in einer geeigneten Nährlösung erhaltene Suspension mit α- oder β-Interferon vorbehandelt, mit einem α-Interferon-Induktor in Berührung bringt, die α-interferonhaltige Flüssigkeit von den Zellen abtrennt, das α-Interferon erwünschtenfalls gewinnt, die Zellen wäscht und in einer geeigneten Nährlösung suspendiert, mit einem mitogenen Mittel behandelt, die γ-interferonhaltige Flüssigkeit von den Zellen abtrennt und erwünschtenfalls das γ-Interferon gewinnt und erwünschtenfalls α-interferonfrei macht.

2. Verfahren nach Anspruch 1, d a d u r c h g e k e n n z e i c h n e t , dass man die Vorbehandlung mit 10-500 IE/ml , vorzugsweise 200-300 IE/ml, α- oder β-Interferon für eine Zeitdauer von 1-6 Stunden lang, vorzugsweise während 2 Stunden, bei 35-39°C, vorzugsweise bei 37°C, durchführt.

3. Verfahren nach Anspruch 1, d a d u r c h g e k e n n z e i c h n e t , dass man als α-Interferon-Induktor rohen oder gereinigten Sendai-Virus verwendet.

4. Verfahren nach Anspruch 3, d a d u r c h g e k e n n z e i c h n e t , dass man den Sendai--Virus in einer Konzentration von 1C0-8C0 , vorzugsweise 400 hemagglutinierenden Einheiten/ml , verwendet.

5. Verfahren nach Anspruch 3 oder 4, d a d u r c h g e k e n n z e i c h n e t , dass man die $\alpha$-Interferon-Induktion 5-48 Stunden lang , vorteilhaft 15-20 Stunden lang, bei einer Temperatur von 35-39°C, vorteilhaft bei 37°C, durchführt.

6. Verfahren nach Anspruch 1, d a d u r c h g e k e n n z e i c h n e t , dass man als mitogenes Mittel Concanavalin A, Phytohaemagglutinin oder Staphylococcus enterotoxin verwendet.

7. Verfahren nach Anspruch 6, d a d u r c h g e k e n n z e i c h n e t , dass man die $\gamma$-Interferon-Induktion 8-43 Stunden lang, vorteilhaft in 12-26 Stunden , durchführt.

8. Verfahren nach einem der Ansprüche 1-7, d a d u r c h g e k e n n z e i c h n e t , dass man das erhaltene rohe $\gamma$-Interferon teilweise reinigt und konzentriert und das anwesende $\alpha$-Interferon durch Gelfiltrieren oder Bindung mit anti-$\alpha$-Interferon-Antikörpern entfernt.

9. Verfahren nach einem der Ansprüche 1-8, d a d u r c h g e k e n n z e i c h n e t , dass man aus der "Buffy coat" - Fraktion die Erythrozyten durch Haemolyse mit Ammoniumchlorid entfernt.

10. Verfahren nach einem der Ansprüche 1-9, d a d u r c h g e k e n n z e i c h n e t , dass man als Nährlösung eine 175-350 mg/l Calciumchlorid, 300-500 mg/l Kaliumchlorid, 175-500 mg/l Magnesiumsulfat oder eine äquivalente Menge von Magnesiumchlorid, 5000-7000 mg/l Natriumchlorid, 200-3500 mg/l Natriumbicarbonat, 30-150 mg/l Natriumdihydrogenphosphat, 500-5500 mg/l Glukose und gegebenenfalls 0,0-0,2 mg/ml Eisen-/III/-nitrat und 0,5-10 % Serum oder Serumprotein (0,5-5 mg/ml) enthaltende wässrige Nährlösung verwendet.

FIG.1

Bestimmung des IFNγ —Gehaltes der aus IFNα und IFNγ bestehenden Mischung

·Kalibrationskurve·

$\frac{IFN\alpha\ +\ IFN\gamma}{IFN\alpha-Titer(\,pH\ 2\ behandelt\ IFN\alpha\ +\ IFN\gamma\,)}$

$\log\frac{IFN\gamma-Titer\ (\,gesucht\,)}{IFN\alpha-Titer(\,pH\ 2\ behandelt\ IFN\alpha\ +\ IFN\gamma\,)}$

1/1 . 0146107